Europäisches Patentamt

European Patent Office

Office européen des brevets

(19)

(11) Veröffentlichungsnummer: **0 175 891**
**B1**

(12) **EUROPÄISCHE PATENTSCHRIFT**

(45) Veröffentlichungstag der Patentschrift:
**13.07.88**

(21) Anmeldenummer: **85109775.8**

(22) Anmeldetag: **03.08.85**

(51) Int. Cl.⁴: **C 07 C 69/82,** C 07 C 67/52

(54) Verfahren zur Abtrennung von Dimethylisophthalat und Dimethylorthophthalat aus ihrem Gemisch mit Dimethylterephthalat.

(30) Priorität: **20.08.84 DE 3430555**

(43) Veröffentlichungstag der Anmeldung:
**02.04.86 Patentblatt 86/14**

(45) Bekanntmachung des Hinweises auf die Patenterteilung:
**13.07.88 Patentblatt 88/28**

(84) Benannte Vertragsstaaten:
**BE DE FR GB IT NL**

(56) Entgegenhaltungen:
**DE - A - 2 323 219**
**US - A - 3 600 430**
**US - A - 3 621 664**

(73) Patentinhaber: **HOECHST AKTIENGESELLSCHAFT,
Postfach 80 03 20, D-6230 Frankfurt am Main 80 (DE)**

(72) Erfinder: **Bader, Rolf, Dr., Pestalozzistrasse 39,
D-6057 Dietzenbach (DE)**
Erfinder: **Last, Hartmut, Dr., Am Hirtenschild 29,
D-6050 Offenbach/Main (DE)**
Erfinder: **Mayer, Manfred, Dr., Königsberger Strasse 8,
D-6272 Niedernhausen/Taunus (DE)**
Erfinder: **Rittner, Siegbert, Dr., Kornblumenweg 5,
D-6105 Mörfelden-Walldorf (DE)**
Erfinder: **Wetzel, Edgar, Dr., Breslauer Strasse 60,
D-6056 Heusenstamm (DE)**

## Beschreibung

Die Erfindung betrifft ein Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol oder dessen Gemisch mit p-Tolnylsäuremethylester (PTE) und anschliessender Veresterung mit Methanol gebildet wird.

Bei diesem grosstechnisch bedeutendsten DMT-Prozess wird zunächst eine durch Kobaltsalz katalysierte Flüssigphasen-Oxidation des p-Xylolos mit sauerstoffhaltigen Gasen durchgeführt. p-Xylol wird dabei mit einem Reingehalt von über 99% eingesetzt. Die Verunreinigungen (weniger als 1%) von p-Xylol bestehen u.a. aus den Isomeren m-Xylol und o-Xylol in wechselnden Mengen. Daher enthält das hergestellte DMT entsprechende Mengen der Isomeren DMI und DMO.

Die Oxidation des p-Xylol bleibt auf der Stufe der p-Toluylsäure stehen. Deren (eine) Carboxylgruppe wird mit Methanol verestert, wobei p-Toluylsäuremethylester (PTE) entsteht. Dann wird dessen Methylgruppe zur Carboxylgruppe oxidiert und anschliessend diese verestert, wobei dann das DMT entsteht.

Man kann die beiden Oxidationsstufen zusammenfassen und die beiden Veresterungsstufen ebenfalls (Witten, Hercules, California Research): Man oxidiert ein Gemisch aus p-Xylol und p-Toluylsäuremethylester (PTE), und das entstehende Gemisch aus p-Toluylsäure und Terephthalsäuremonomethylester wird dann verestert zu p-Toluylsäuremethylester (PTE) und Dimethylterephthalat (DMT). Die beiden Ester werden destillativ getrennt. Der PTE wird in die Oxidation zurückgeführt, während das DMT weiter gereinigt wird.

Man kann aber auch beide Oxidationen und beide Veresterungen in einer Stufe zusammenfassen (BASF, Du Pont und Montecatini). Das als Lösemittel für die Oxidation dienende Methanol verestert die oxidierten Methylgruppen anschliessend im selben Reaktor. Dieser arbeitet nach dem Gegenstromprinzip, dabei werden von oben p-Xylol und zurückgeführte Teiloxidationsprodukte und von unten Methanol und Luft eingespeist. Bei 100–200 °C und 5–20 bar wird bei 22 Stunden Verweilzeit die Oxidation mit Hilfe von Kobalt-Salzen durchgeführt.

Nach beiden Methoden erhält man ein rohes DMT, das destilliert und umkristallisiert werden muss, um reines DMT zu erhalten.

Bei beiden Methoden bilden sich aber auch die DMT-Isomeren Dimethylisophthalat (DMI) und Dimethylorthophthalat (DMO) gemäss dem erwähnten Anteil an o-Xylol und m-Xylol im eingesetzten p-Xylol.

Beide Male erhebt sich daher das Problem der Ausschleusung von DMI und DMO, das am Beispiel des oben erwähnten Witten-Prozesses exemplarisch dargestellt werden soll.

Ein Gemisch aus p-Xylol und PTE wird in flüssiger Phase unter Druck und bei 140–170 °C mit Luft in Gegenwart eines Kobalt enthaltenden Katalysators oxidiert. Das Oxidationsprodukt besteht aus p-Toluylsäure und Monomethylterephthalat sowie etwas Terephthalsäure. Das Oxidationsprodukt wird anschliessend mit Methanol verestert, z.B. bei 240 °C und 30–40 bar, und dann in einer sogenannten Rohesterdestillation in eine PTE-reiche Fraktion, eine DMT-reiche Fraktion sowie in einen Destillationsrückstand getrennt, der die hochsiedenden Nebenprodukte des Verfahrens enthält (z.B. Diphenylkörper). Die PTE-reiche Fraktion wird in die Oxidation zurückgeführt. Die DMT-reiche Fraktion wird nachfolgenden Reinigungen zugeführt, die in der Regel aus einer Rektifikation und zwei Umkristallisationen aus Methanol bestehen. Da sich die in dieser Fraktion enthaltenen Isomeren DMI und DMO nur wenig in ihren Siedepunkten vom DMT unterscheiden, gelingt eine Trennung vom DMT erst durch die Umkristallisation aus Methanol. Dabei gelangen DMI und DMO in die Mutterlauge, worin ausserdem gelöst sind: restliches DMT, restlicher PTE (der in der Rohesterdestillation unvollständig abgetrennt wurde) und weitere Teiloxidationsprodukte, wie 4-Carbomethoxybenzaldehyd und dessen Dimethylacetal.

p-Toluylester (PTE), 4-Carbomethoxybenzaldehyd und sein Dimethylacetal sind DMT-Vorstufen. Deshalb wird der nach dem Abdampfen des Methanols von der Mutterlauge verbleibende Rückstand (im folgenden auch kurz als «Rückstand der Mutterlauge» oder als «eingedampfte Mutterlauge» bezeichnet) in die Oxidation zurückgeführt. Hiermit werden aber zwangsläufig gleichzeitig Stoffe zurückgeführt, die keine DMT-Vorstufen sind, sondern bereits zweimal oxidiert und verestert sind, nämlich DMT selbst und seine beiden Isomeren. Diese Stoffe sind daher als Ballast des Prozesses anzusehen. Da DMT bei der Umkristallisation dauernd als gewünschtes Produkt ausgeschleust wird, steigt sein Anteil trotz der Rückführung nicht über einen bestimmten Pegel. Dagegen verbleiben DMI und DMO bei der Umkristallisation als unerwünschte Stoffe in der Mutterlauge und reichern sich immer mehr an, solange sie nicht irgendwie ausgeschleust werden. Sie beanspruchen dann im Laufe der Jahre stetig wachsende Anteile in den Produktionsströmen. Nicht nur eine Überlastung der Reaktoren und Rektifikationen ist die Folge, sondern auch eine zunehmende Ineffizienz der Umkristallisation, die sich darin zeigt, dass der überwiegende Anteil der im umkristallisierten DMT verbliebenen Verunreinigungen aus DMI und DMO besteht. Das der Umkristallisation zugeführte «rohe DMT» kann dann DMI-DMO-Gehalte von 20 Gew.-% haben, wobei der entsprechende Gehalt in der Mutterlauge bis zu 50 Gew.-% beträgt.

Um den DMI-DMO-Pegel in der Anlage nicht allzu hoch steigen zu lassen, muss man ab einem bestimmten Punkt diejenige Menge an DMI und DMO ausschleusen, die sich in Abhängigkeit mit Isomerengehalt des eingesetzten p-Xylols in der Anlage dauernd neu bildet. In der Praxis kann man dies z.B. dadurch erreichen, dass man einen Teil der eingedampften Mutterlauge der Umkristallisation verwirft. Dabei müssen jedoch DMT-Verluste in Kauf genommen werden, die umso höher sind, je niedriger man den DMI-DMO-Pegel in der Anlage halten will. Bei einem DMI-DMO-Gehalt von 50 Gew.-% in der Mutterlauge werden je kg DMI-DMO auch 1 kg DMT (und Vorstufen) verworfen, was gerade noch vertretbar erscheinen mag. Soll ein niedrigerer DMI-DMO-Pegel in der Anlage gehalten werden, so ist die Anreicherung von DMI und DMO in der Mutterlauge der Umkristallisation natürlich entsprechend niedriger. Die Ausschleusung von DMI und DMO durch Verwerfen von eingedampfter Mutterlauge wird also mit abnehmendem DMI-DMO-Pegel immer verlustreicher und schliesslich untragbar: Während im oben erwähnten Fall eines DMI-DMO-Gehaltes von 20 Gew.-% im umzukristallisierenden DMT eine Ausbeuteminderung von ca. 0,1% eintritt, müssten bei einem niedrigeren DMI-DMO-Gehalt von 5 Gew.-% im umzukristallisierenden DMT ca. 0,7% Ausbeuteverlust in Kauf genommen werden. Damit die DMI-DMO-Ausschleusung nicht allzu verlustreich ist, muss man daher einen hohen DMI-DMO-Pegel einhalten, was aber einen hohen Ballastanteil im Kreislauf bedeutet.

Die vorliegende Erfindung betrifft eine auch bei niedrigem DMI-DMO-Pegel verlustarme Ausschleusung von DMI und DMO. Die Ausbeuteverluste liegen je nach Isomerengehalt des eingesetzten p-Xylols bei nur 0,05%, praktisch unabhängig vom DMI-DMO-Pegel in der Anlage.

Auf diese Weise kann der Ballast an DMI und DMO in der Anlage weitgehend reduziert werden, so dass eine volle Nutzung der Produktionskapazität möglich wird.

Bei dem erfindungsgemässen Verfahren gewinnt man ein DMI-DMO-Konzentrat, indem man die eingedampfte Mutterlauge (der Methanol-Umkristallisation) bzw. einen Teil davon, oder die DMI-reiche Fraktion der oben erwähnten Rohesterdestillation bzw. einen Teil dieser Fraktion einer ein- oder mehrmaligen Schmelzkristallisation unterwirft. Das DMI-DMO-Konzentrat hat vorzugsweise fast eutektische Zusammensetzung und daher einen niedrigen DMT-Gehalt und kann daher ausgeschleust werden, ohne dass wesentliche DMT-Verluste eintreten.

Aus DE-A-2 323 219 ist ein Verfahren zur Reinigung von rohem DMT durch Schmelzkristallisation in Gegenwart eines Lösungsmittels bekannt, das zur Abtrennung der Verunreinigungen dient. Überraschenderweise kommt das erfindungsgemässe Verfahren ohne Lösungsmittel zum Erfolg.

Ein Gegenstand der vorliegenden Erfindung ist ein Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol oder dessen Gemisch mit PTE und anschliessender Veresterung mit Methanol gebildet wird, wobei man

a) das Veresterungsprodukt ein oder mehrmals destilliert,
b) die in Schritt a) erhaltene DMT-reiche Fraktion aus Methanol umkristallisiert,

c) das in Schritt b) erhaltene kristalline DMT abzieht,
d) aus der Mutterlauge von Schritt b) das Methanol abdampft,
dadurch gekennzeichnet, dass man
e) den in Schritt d) erhaltenen hauptsächlich aus DMT, DMI und DMO bestehenden Rückstand schmilzt und durch Abkühlen DMT auskristallisieren lässt, ohne mit dem DMT-Gehalt in den Nebenprodukten die eutektische Zusammensetzung zu erreichen,
f) das in Schritt e) erhaltene DMT abzieht,
g) die in Schritt e) erhaltene DMI-DMO-Fraktion zumindest teilweise ausschleust.

Nach dieser Methode wird also der gesamte Rückstand der Mutterlauge der ein- oder mehrmaligen Schmelzkristallisation unterworfen.

In Schritt a) wird vorzugsweise zweimal destilliert. Bei der ersten Destillation (Rohesterdestillation) werden p-Toluylsäuremethylester (PTE) und Hochsieder vom DMT abgetrennt; der PTE wird in die Oxidationsstufe zurückgeführt. Bei der zweiten Destillation wird die so erhaltene DMT-reiche Fraktion rektifiziert.

In Schritt b) wird im allgemeinen zweimal aus Methanol umkristallisiert.

In Schritt e) geht man vorzugsweise möglichst nahe an den eutektischen Punkt heran (ohne ihn allerdings zu erreichen), damit die Nebenprodukte, insbesondere DMI und DMO, möglichst hoch angereichert werden. Da die Nebenprodukte dann fast eutektische Zusammensetzung haben, enthalten sie nur sehr wenig DMT.

Im einzelnen geht man wie folgt vor: der Rückstand der Mutterlauge wird geschmolzen, und dann vorzugsweise innerhalb von 0,5 bis 2 Stunden zunächst auf eine Temperatur abgekühlt, die 0,5 bis 15 °C unter dem Erstarrungspunkt des Ausgangsgemischs liegt.

Die optimale Temperatur, auf die hierbei abgekühlt wird, hängt von der Art und Menge der diversen Verunreinigungen, aber auch vom verwendeten Kristallisationsapparat ab. Bei der gewählten Temperatur lässt man die Kristalle je nach verwendetem Kristallisationsapparat wenige Minuten bis wenige Stunden wachsen, dann kühlt man weiter ab, vorzugsweise bis in die Nähe der eutektischen Temperatur, ohne sie jedoch zu erreichen. Insbesondere kühlt man bis auf eine Temperatur ab, die nur 0,5 bis 3 °C über der eutektischen Temperatur liegt. Als eutektische Temperatur wird dabei die Temperatur bezeichnet, bei der sich das jeweilige, von der Art der Verunreinigung des DMT abhängige eutektische Gemisch abscheidet. Der flüssig verbliebene Anteil wird von den Kristallen abgetrennt, die in und an den Kristallen anhaftenden flüssigen Verunreinigungen durch möglichst gleichmässige Temperaturerhöhung abgeschieden und das so gereinigte DMT durch weiteres Aufschmelzen gewonnen.

Bei der Reinigung in einem statischen Platten- oder Röhrenkristallisator (Tropfapparat), wie in Winnacker-Küchler, Chemische Technologie, 4. Auflage, Band 6, 1982, Seite 148, beschrieben, kühlt man zunächst vorzugsweise nur auf 1,5 bis 3 °C unter den Erstarrungspunkt des Ausgangsgemischs und hält diese Temperatur 1–3 Stunden aufrecht, um eine gute Ausbildung der Kristalle zu erreichen.

Dann kühlt man, vorzugsweise innerhalb von 2 bis 20, insbesondere 4 bis 10 Stunden, weiter ab, vorzugsweise bis in die Nähe der eutektischen Temperatur. Die dann im Kristallisator noch flüssig gebliebenen Verunreinigungen werden abgetrennt, und die verbleibende Kristallmasse wird weiter durch Temperaturerhöhung gereinigt.

Diese Temperaturerhöhung soll langsam stattfinden, vorzugsweise mit einer Steigerung von ca. 0,5 bis 2 °C pro Stunde. Die am Anfang dieses Schmelzvorgangs anfallenden flüssigen Fraktionen werden abgetrennt und einer erneuten Schmelzkristallisation unterworfen. Die im Apparat schliesslich verbliebene Kristallmasse stellt das isolierte DMT dar.

Arbeitet man ein- oder (bevorzugt) mehrstufig mit dem in der US-PS 3 621 664 beschriebenen Rieselfilmkristallisator, so kühlt man hier die Gemische zunächst vorzugsweise für wenige Minuten auf eine Temperatur von ca. 1 bis 10 °C unter den Erstarrungspunkt des Ausgangsgemischs. Dann wird vorzugsweise innerhalb von 0,5 bis 3 Stunden, insbesondere innerhalb von 0,5 bis 1,5 Stunden weiter abgekühlt, vorzugsweise wieder bis in die Nähe der eutektischen Temperatur. Dann wird vom verbleibenden flüssigen Anteil abgetrennt. Die weitere Reinigung erfolgt dann durch einen Schwitzvorgang der Kristalle bei ansteigender Temperatur. Das so isolierte Dimethylterephthalat wird dann durch weiteres Erwärmen aufgeschmolzen und aus dem Apparat abgelassen, oder ggf. einer erneuten Schmelzkristallisation unterworfen.

Die optimalen Temperaturen für die Auslösung der Kristallisation, die Erzielung eines guten Kristallwachstums zwecks wirksamer Abtrennung der Verunreinigungen, sowie die günstigsten Kristallisationszeiten lassen sich für jedes Ausgangsgemisch in einem Vorversuch an Hand der obigen Erläuterungen leicht ermitteln.

Da das Ziel von Schritt e) die möglichst hohe Konzentration von DMI und DMO und die möglichst niedrige Konzentration von DMT in der Nebenprodukt-Fraktion (DMI-DMO-Fraktion) der Schmelzkristallisation ist, enthält das auskristallisierende DMT im allgemeinen noch Reste an Nebenprodukten. Es wird daher im allgemeinen anschliessend aus Methanol umkristallisiert, und zwar vorzugsweise gemeinsam mit der DMT-reichen Fraktion aus Schritt a).

Der etwaige in Schritt g) nicht ausgeschleuste Anteil der DMI-DMO-Fraktion wird wegen seines Gehaltes an teiloxidierten DMT-Vorstufen (4-Carbomethoxybenzaldehyd und sein Dimethylacetal sowie p-Toluylester) in die Oxidationsstufe zurückgeführt. Vorzugsweise werden nur etwa 5–15 Gew.-% der DMI-DMO-Fraktion ausgeschleust und der Rest zurückgeführt. Man kann jedoch diesen Rest noch destillieren und dann nur die teiloxidierten DMT-Vorstufen zurückführen.

Statt des gesamten Rückstands der Mutterlauge (der Methanol-Umkristallisation) kann man auch einen Teil davon der ein- oder mehrmaligen Schmelzkristallisation unterwerfen. Ein weiterer Gegenstand der Erfindung ist daher ein Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol oder dessen Gemisch mit PTE und anschliessender Veresterung mit Methanol gebildet wird, wobei man

a) das Veresterungsprodukt ein oder mehrmals destilliert,

b) die in Schritt a) erhaltene DMT-reiche Fraktion aus Methanol umkristallisiert,

c) das in Schritt b) erhaltene kristalline DMT abzieht,

d) aus der Mutterlauge von Schritt b) das Methanol abdampft, dadurch gekennzeichnet, dass man

e) einen Teil des in Schritt d) erhaltenen hauptsächlich aus DMT, DMI und DMO bestehenden Rückstands schmilzt und durch Abkühlen DMT auskristallisieren lässt, ohne mit dem DMT-Gehalt in den Nebenprodukten die eutektische Zusammensetzung zu erreichen,

f) das in Schritt e) erhaltene DMT abzieht,

g) die in Schritt e) erhaltene DMI-DMO-Fraktion ausschleust,

h) den nicht in Schritt e) eingesetzten Teil des in Schritt d) erhaltenen Rückstands in die Oxidationsstufe zurückführt.

Bei dieser Methode wird also ein Teil des Rückstandes der Mutterlauge gemäss dem Stand der Technik in die Oxidation zurückgeführt, und der andere Teil wird zur Ausschleusung von DMI und DMO durch Schmelzkristallisation verwendet. Hiermit verringern sich die Kapazitäten der benötigten Schmelzkristallisationsapparate gegenüber der vorher beschriebenen Methode.

Für die Schritte a), b) und e) gilt wieder das bereits bei der ersten Methode gesagte, nur dass jetzt in Schritt e) lediglich ein Teil des Rückstands der Mutterlauge eingesetzt wird. Der nicht hier eingesetzte Teil wird gemäss Schritt h) in die Oxidationsstufe zurückgeführt.

Wieviel von dem Rückstand man in Schritt e) einsetzt, hängt ab von der Neubildungsrate an DMI und DMO, welche wiederum determiniert wird durch den Isomerengehalt des eingesetzten p-Xylols. Bei einer p-Xylol-Qualität von über 99% wie sie üblicherweise auf dem Weltmarkt gehandelt wird, setzt man vorzugsweise 5–15 Gew.-% des Rückstandes der Mutterlauge in Schritt e) ein, wenn man einen Pegel von ca. 3–5% an DMI und DMO in der DMT-reichen Fraktion von Schritt b) halten will. Wenn man einen geringeren Anteil des Rückstandes der Mutterlauge in Schritt e) einsetzt, werden sich entsprechend höhere DMI-DMO-Pegel einstellen.

Die Mutterlauge der Methanol-Umkristallisation ist dasjenige Gemisch in der Anlage, welches die höchste Konzentration an DMI und DMO hat. Daher wird vorzugsweise der Rückstand der Mutterlauge zur Ausschleusung von DMI und DMO herangezogen. Darauf basieren die beiden soeben beschriebenen Methoden. Aber auch die DMT-reiche Fraktion, die nach dem Stand der Technik und auch nach den beiden obigen Methoden (jeweils Schritt b)) aus Methanol umkristallisiert wird, hat eine relativ hohe DMI-DMO-Konzentration und kann daher zur Ausschleusung dieser Stoffe herangezogen werden. Man kann entweder die gesamte DMT-reiche Fraktion oder einen Teil davon der ein- oder mehrmaligen Schmelzkristallisation zuführen. Ein weiterer Gegenstand der Erfindung ist daher ein Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol oder dessen Gemisch mit PTE und anschliessender Veresterung mit Methanol gebildet wird, wobei man

a) das Veresterungsprodukt ein- oder mehrmals destilliert, dadurch gekennzeichnet, dass man

b) die in Schritt a) erhaltene DMT-reiche Fraktion schmilzt und durch Abkühlen DMT auskristallisieren lässt, ohne mit dem DMT-Gehalt in den Nebenprodukten die eutektische Zusammensetzung zu erreichen,

c) das in Schritt b) erhaltene DMT aus Methanol umkristallisiert,

d) die im Schritt b) erhaltene DMI-DMO-Fraktion zumindest teilweise ausschleust,

e) das in Schritt c) erhaltene kristalline DMT abzieht,

f) aus der Mutterlauge von Schritt c) das Methanol abdampft und den Rückstand in die Oxidationsstufe zurückführt.

Das in Schritt b) erhaltene DMT ist bereits relativ rein, so dass die Umkristallisation aus Methanol in Schritt c) wesentlich erleichtert wird. Meist genügt hier schon einmalige Umkristallisation, während nach dem Stand der Technik eine zweimalige Umkristallisation erforderlich war.

Der etwaige in Schritt d) nicht ausgeschleuste Anteil der DMI-DMO-Fraktion wird in die Oxidationsstufe zurückgeführt. Vorzugsweise werden nur etwa 5–15 Gew.-% ausgeschleust und der Rest zurückgeführt.

Für die Schmelzkristallisation, die hier als Schritt b) durchgeführt wird, gilt das zu diesem Thema bei den beiden ersten Methoden gesagte (wo die Schmelzkristallisation jeweils den Schritt e) bildet).

Statt der gesamten DMT-reichen Fraktion aus der Destillation des Veresterungsprodukts kann man auch einen Teil davon der Schmelzkristallisation unterwerfen. Ein weiterer Gegenstand der Erfindung ist daher ein Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol oder dessen Gemisch mit PTE und anschliessender Veresterung mit Methanol gebildet wird, wobei man

a) das Veresterungsprodukt ein- oder mehrmals destilliert, dadurch gekennzeichnet, dass man

b) einen Teil der in Schritt a) erhaltene DMT-reichen Fraktion schmilzt und durch Abkühlen DMT auskristallisieren lässt, ohne mit dem DMT-Gehalt in den Nebenprodukten die eutektische Zusammensetzung zu erreichen,

c) das in Schritt b) erhaltene DMT aus Methanol umkristallisiert,

d) die im Schritt b) erhaltene DMI-DMO-Fraktion ausschleust,

e) das in Schritt c) erhaltene kristalline DMT abzieht,

f) aus der Mutterlauge von Schritt c) das Methanol abdampft und den Rückstand in die Oxidationsstufe zurückführt.

g) den nicht in Schritt b) eingesetzten Teil der in Schritt a) erhaltenen DMT-reichen Fraktion aus Methanol umkristallisiert, das so erhaltene kristalline DMT abzieht, aus der Mutterlauge das Methanol abdampft und den Rückstand in die Oxidationsstufe zurückführt.

Für Schritt a) gilt wieder das bereits gesagte. Ebenso für die als Schritt b) durchgeführte Schmelzkristallisation. In Schritt c) genügt im allgemeinen wieder eine einmalige Umkristallisation. Vorzugsweise werden 5–15 Gew.-% der in Schritt a) erhaltenen DMT-reichen Fraktion in Schritt b) eingesetzt. Der Rest wird in Schritt g) benutzt. In Schritt g) wird vorzugsweise zweimal umkristallisiert.

Die Erfindung wird durch folgende Beispiele erläutert. Die Prozentzahlen sind jeweils Gewichtsprozente.

Beispiel 1

In einem Röhrenkristallisator, dessen Mantel an einen Thermostaten und einen Temperatur-Zeitplangeber angeschlossen ist, werden 532 g eines methanolfreien Rückstands aus der Umkristallisation von DMT mit Methanol mit einem Erstarrungspunkt von 87 °C und folgender Zusammensetzung eingesetzt:

Dimethylterephthalat (DMT): 29,0%
Dimethylisophthalat (DMI): 56,3%
Dimethylorthophthalat (DMO): 4,7%
4-Carbomethoxybenzaldehyd-dimethylacetal: 3,0%
4-Carbomethoxybenzaldehyd: 1,1%
p-Toluylsäuremethylester (PTE): 5,8%
Benzoesäuremethylester: 0,1%

Man kühlt das Gemisch auf 85, 5 °C ab, wobei die Kristallisation rasch einsetzt. Nach etwa einer Stunde bei 84–85,5 °C wird innerhalb von 12 Stunden auf 30 °C abgekühlt und danach der Kristallisator geöffnet. Man lässt den flüssig verbliebenen Anteil ablaufen und erhöht langsam die Temperatur der im Kristallisator verbliebenen Kristallmasse auf 68 °C, wobei man die hierbei abtropfende Schmelze mit der bereits bei 30 °C abgelaufenen Schmelze vereint. Man erhält 140 g Kristallisat mit einem Erstarrungspunkt von 133,4 °C und 390 g Kristallisationsrückstand mit einem Erstarrungspunkt von 64,5 °C. Dieser Rückstand hatte die folgende Zusammensetzung:
Dimethylterephthalat (DMT): 14,5%
Dimethylisophthalat (DMI): 64,0%
Dimethylorthophthalat (DMO): 8,1%
4-Carbomethoxybenzaldehyd-dimethylacetal: 4,9%
4-Carbomethoxybenzaldehyd: 2,01%
p-Toluylsäuremethylester (PTE): 6,4%
Benzoesäuremethylester: 0,1%

Beispiel 2

In einem Kristallisator wie in der US-Patentschrift 3 621 664 beschrieben, wurden zur Schmelzkristallisation 29,8 kg eines methanolfreien Rückstands aus der Umkristallisation von DMT mit Methanol mit einem Erstarrungspunkt von 89,2 °C und folgender Zusammensetzung eingesetzt:
Dimethylterephthalat (DMT): 30,7%
Dimethylisophthalat (DMI): 54,1%
Dimethylorthophthalat (DMO): 5,6%
4-Carbomethoxybenzaldehyd-dimethylacetal: 3,2%
4-Carbomethoxybenzaldehyd: 1,1%
p-Toluylsäuremethylester (PTE): 5,2%
Benzoesäuremethylester: 0,1%
Dieses Gemisch wurde bis 20 °C (Kühlmediumtemperatur) während 100 Minuten kristallisiert. Hierbei wurde ein Kristallisat (7,9 kg) mit einem Erstarrungspunkt von 124 °C und ein Kristallisationsrückstand (21,9 kg) mit einem Erstarrungspunkt von 63 °C erhalten. Dieser Rückstand hatte die folgende Zusammensetzung:
Dimethylterephthalat (DMT): 15,15%
Dimethylisophthalat (DMI): 60,8%
Dimethylorthophthalat (DMO): 9,6%
4-Carbomethoxybenzaldehyd-dimethylacetal: 5,5%
4-Carbomethoxybenzaldehyd: 2,03%
p-Toluylsäuremethylester (PTE): 6,82%
Benzoesäuremethylester: 0,1%

Beispiel 3

In einem Kristallisator wie in der US-Patentschrift 3 621 664 beschrieben, wurden zur Schmelzkristallisation 20 kg Kristallisationsrückstand mit einem Erstarrungspunkt von 63 °C aus der Kristallisation von Beispiel 2 eingesetzt.
Dieses Gemisch wurde im Temperaturbereich bis 0 °C (Kühlmediumtemperatur) während 120 Minuten kristallisiert. Hierbei wurden 5,8 kg Kristallisat mit einem Erstarrungspunkt von 106 °C und 14,2 kg Kristallisationsrückstand mit einem Erstarrungspunkt von 49 °C erhalten. Der Gehalt an DMT in diesem Rückstand (mit den Begleitstoffen Dimethylisophthalat, Dimethylorthophthalat, 4-Carbomethoxybenzaldehyd-dimethylacetal, 4-Carbomethoxybenzaldehyd und p-Toluylsäuremethylester) lag nur noch bei ca. 10%.

**Patentansprüche**

1. Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol und anschliessender Veresterung mit Methanol gebildet wird, wobei man
a) das Veresterungsprodukt ein- oder mehrmals destilliert,
b) die in Schritt a) erhaltene DMT-reiche Fraktion aus Methanol umkristallisiert,
c) das in Schritt b) erhaltene kristalline DMT abzieht,
d) aus der Mutterlauge von Schritt b) das Methanol abdampft,
dadurch gekennzeichnet, dass man
e) den in Schritt d) erhaltenen hauptsächlich aus DMT, DMI und DMO bestehenden Rückstand schmilzt und durch Abkühlen DMT auskristallisieren lässt, ohne mit dem DMT-Gehalt in den Nebenprodukten die eutektische Zusammensetzung zu erreichen,
f) das in Schritt e) erhaltene DMT abzieht,
g) die in Schritt e) erhaltene DMI-DMO-Fraktion zumindest teilweise ausschleust.
2. Verfahren nach Anspruch 1, dadurch gekennzeichnet, dass man in Schritt e) den geschmolzenen Rückstand zunächst innerhalb von 0,5 bis 2 Stunden auf eine Temperatur abkühlt, die 0,5 bis 15 °C unter dem Erstarrungspunkt des Gemischs liegt, dann die Kristalle wachsen lässt, und dann weiter abkühlt, ohne jedoch die eutektische Temperatur zu erreichen.
3. Verfahren nach Anspruch 1 oder 2, dadurch gekennzeichnet, dass man in Schritt e) bis auf eine Temperatur abkühlt, die nur 0,5 bis 3 °C über der eutektischen Temperatur liegt.
4. Verfahren nach einem der Ansprüche 1 bis 3, dadurch gekennzeichnet, dass man in Schritt g) 5–15 Gew.-% der DMI-DMO-Fraktion ausschleust und den Rest in die Oxidationsstufe zurückführt.
5. Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol und anschliessender Veresterung mit Methanol gebildet wird, wobei man
a) das Veresterungsprodukt ein- oder mehrmals destilliert,
b) die in Schritt a) erhaltene DMT-reiche Fraktion aus Methanol umkristallisiert,
c) das in Schritt b) erhaltene kristalline DMT abzieht,
d) aus der Mutterlauge von Schritt b) das Methanol abdampft,
dadurch gekennzeichnet, dass man
e) einen Teil des in Schritt d) erhaltenen hauptsächlich aus DMT, DMI und DMO bestehenden Rückstands schmilzt und durch Abkühlen DMT auskristallisieren lässt, ohne mit dem DMT-Gehalt in den Nebenprodukten die eutektische Zusammensetzung zu erreichen,

f) das in Schritt e) erhaltene DMT abzieht,

g) die in Schritt e) erhaltene DMI-DMO-Fraktion ausschleust,

h) den nicht in Schritt e) eingesetzten Teil des in Schritt d) erhaltenen Rückstands in die Oxidationsstufe zurückführt.

6. Verfahren nach Anspruch 5, dadurch gekennzeichnet, dass man in Schritt e) den geschmolzenen Teil des Rückstands zunächst innerhalb von 0,5 bis 2 Stunden auf eine Temperatur abkühlt, die 0,5 bis 15 °C unter dem Erstarrungspunkt des Gemisches liegt, dann die Kristalle wachsen lässt, und dann weiter abkühlt, ohne jedoch die eutektische Temperatur zu erreichen.

7. Verfahren nach Anspruch 5 oder 6, dadurch gekennzeichnet, dass man in Schritt e) bis auf eine Temperatur abkühlt, die nur 0,5 bis 3 °C über der eutektischen Temperatur liegt.

8. Verfahren nach einem der Ansprüche 5 bis 7, dadurch gekennzeichnet, dass man 5–15 Gew.-% des in Schritt d) erhaltenen Rückstands in Schritt e) einsetzt und den Rest in die Oxidationsstufe zurückführt.

9. Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol und anschliessender Veresterung mit Methanol gebildet wird, wobei man

a) das Veresterungsprodukt ein- oder mehrmals destilliert, dadurch gekennzeichnet, dass man

b) die in Schritt a) erhaltene DMT-reiche Fraktion schmilzt und durch Abkühlen DMT auskristallisieren lässt, ohne mit dem DMT-Gehalt in den Nebenprodukten die eutektische Zusammensetzung zu erreichen,

c) das in Schritt b) erhaltene DMT aus Methanol umkristallisiert,

d) die im Schritt b) erhaltene DMI-DMO-Fraktion zumindest teilweise ausschleust,

e) das in Schritt c) erhaltene kristalline DMT abzieht,

f) aus der Mutterlauge von Schritt c) das Methanol abdampft und den Rückstand in die Oxidationsstufe zurückführt.

10. Verfahren nach Anspruch 9, dadurch gekennzeichnet, dass man in Schritt b) die geschmolzene DMT-reiche Fraktion zunächst innerhalb von 0,5 bis 2 Stunden auf eine Temperatur abkühlt, die 0,5 bis 15 °C unter dem Erstarrungspunkt des Gemischs liegt, dann die Kristalle wachsen lässt, und dann weiter abkühlt, ohne jedoch die eutektische Temperatur zu erreichen.

11. Verfahren nach Anspruch 9 oder 10, dadurch gekennzeichnet, dass man in Schritt b) bis auf eine Temperatur abkühlt, die nur 0,5 bis 3 °C über der eutektischen Temperatur liegt.

12. Verfahren nach einem der Ansprüche 9 bis 11, dadurch gekennzeichnet, dass man in Schritt d) 5–15 Gew.-% der DMI-DMO-Fraktion ausschleust und den Rest in die Oxidationsstufe zurückführt.

13. Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation von p-Xylol und anschliessender Veresterung mit Methanol gebildet wird, wobei man

a) das Veresterungsprodukt ein- oder mehrmals destilliert, dadurch gekennzeichnet, dass man

b) einen Teil der in Schritt a) erhaltenen DMT-reichen Fraktion schmilzt und durch Abkühlen DMT auskristallisieren lässt, ohne mit dem DMT-Gehalt in den Nebenprodukten die eutektische Zusammensetzung zu erreichen,

c) das in Schritt b) erhaltene DMT aus Methanol umkristallisiert,

d) die im Schritt b) erhaltene DMI-DMO-Fraktion ausschleust,

e) das in Schritt c) erhaltene kristalline DMT abzieht,

f) aus der Mutterlauge von Schritt c) das Methanol abdampft und den Rückstand in die Oxidationsstufe zurückführt,

g) den nicht in Schritt b) eingesetzten Teil der in Schritt a) erhaltenen DMT-reichen Fraktion aus Methanol umkristallisiert, das so erhaltene kristalline DMT abzieht, aus der Mutterlauge das Methanol abdampft und den Rückstand in die Oxidationsstufe zurückführt.

14. Verfahren nach Anspruch 13, dadurch gekennzeichnet, dass man in Schritt b) den geschmolzenen Teil der DMT-reichen Fraktion zunächst innerhalb von 0,5 bis 2 Stunden auf eine Temperatur abkühlt, die 0,5 bis 15 °C unter dem Erstarrungspunkt des Gemischs liegt, dann die Kristalle wachsen lässt, und dann weiter abkühlt, ohne jedoch die eutektische Temperatur zu erreichen.

15. Verfahren nach Anspruch 13 oder 14, dadurch gekennzeichnet, dass man in Schritt b) bis auf eine Temperatur abkühlt, die nur 0,5 bis 3 °C über der eutektischen Temperatur liegt.

16. Verfahren nach einem der Ansprüche 13 bis 15, dadurch gekennzeichnet, dass man 5–15 Gew.-% der in Schritt a) erhaltenen DMT-reichen Fraktion in Schritt b) einsetzt und den Rest in die Oxidationsstufe zurückführt.

17. Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation eines Gemisches aus p-Xylol und p-Toluylsäuremethylester und anschliessender Veresterung mit Methanol gebildet wird, wobei man

a) das Veresterungsprodukt ein- oder mehrmals destilliert,

b) die in Schritt a) erhaltene DMT-reiche Fraktion aus Methanol umkristallisiert,

c) das in Schritt b) erhaltene kristalline DMT abzieht,

d) aus der Mutterlauge von Schritt b) das Methanol abdampft, dadurch gekennzeichnet, dass man

e) den in Schritt d) erhaltenen hauptsächlich aus DMT, DMI und DMO bestehenden Rückstand schmilzt und durch Abkühlen DMT auskristallisieren lässt, ohne mit dem DMT-Gehalt in den Nebenprodukten die eutektische Zusammensetzung zu erreichen,
f) das in Schritt e) erhaltene DMT abzieht,
g) die in Schritt e) erhaltene DMI-DMO-Fraktion zumindest teilweise ausschleust.

18. Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation eines Gemischs aus p-Xylol und p-Toluylsäuremethylester und anschliessender Veresterung mit Methanol gebildet wird, wobei man
a) das Veresterungsprodukt ein- oder mehrmals destilliert,
b) die in in Schritt a) erhaltene DMT-reiche Fraktion aus Methanol umkristallisiert,
c) das in Schritt b) erhaltene kristalline DMT abzieht,
d) aus der Mutterlauge von Schritt b) das Methanol abdampft,
dadurch gekennzeichnet, dass man
e) einen Teil des in Schritt d) erhaltenen hauptsächlich aus DMT, DMI und DMO bestehenden Rückstands schmilzt und durch Abkühlen DMT auskristallisieren lässt, ohne mit dem DMT-Gehalt in den Nebenprodukten die eutektische Zusammensetzung zu erreichen,
f) das in Schritt e) erhaltene DMT abzieht,
g) die in Schritt e) erhaltene DMI-DMO-Fraktion ausschleust,
h) den nicht in Schritt e) eingesetzten Teil des in Schritt d) erhaltenen Rückstands in die Oxidationsstufe zurückführt.

19. Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation eines Gemisches aus p-Xylol und p-Toluylsäuremethylester und anschliessender Veresterung mit Methanol gebildet wird, wobei man
a) das Veresterungsprodukt ein- oder mehrmals destilliert,
dadurch gekennzeichnet, dass man
b) die in Schritt a) erhaltene DMT-reiche Fraktion schmilzt und durch Abkühlen DMT auskristallisieren lässt, ohne mit dem DMT-Gehalt in den Nebenprodukten die eutektische Zusammensetzung zu erreichen,
c) das in Schritt b) erhaltene DMT aus Methanol umkristallisiert,
d) die im Schritt b) erhaltene DMI-DMO-Fraktion zumindest teilweise ausschleust,
e) das in Schritt c) erhaltene kristalline DMT abzieht,
f) aus der Mutterlauge von Schritt c) das Methanol abdampft und den Rückstand in die Oxidationsstufe zurückführt.

20. Verfahren zur Abtrennung von Dimethylisophthalat (DMI) und Dimethylorthophthalat (DMO) und anderer Nebenprodukte aus ihrem Gemisch mit Dimethylterephthalat (DMT), das bei der Oxidation eines Gemisches aus p-Xylol und p-Toluylsäuremethylester und anschliessender Veresterung mit Methanol gebildet wird, wobei man
a) das Veresterungsprodukt ein- oder mehrmals destilliert,
dadurch gekennzeichnet, dass man
b) einen Teil der in Schritt a) erhaltenen DMT-reichen Fraktion schmilzt und durch Abkühlen DMT auskristallisieren lässt, ohne mit dem DMT-Gehalt in den Nebenprodukten die eutektische Zusammensetzung zu erreichen,
c) das in Schritt b) erhaltene DMT aus Methanol umkristallisiert,
d) die im Schritt b) erhaltene DMI-DMO-Fraktion ausschleust,
e) das in Schritt c) erhaltene kristalline DMT abzieht,
f) aus der Mutterlauge von Schritt c) das Methanol abdampft und den Rückstand in die Oxidationsstufe zurückführt,
g) den nicht in Schritt b) eingesetzten Teil der in Schritt a) erhaltenen DMT-reichen Fraktion aus Methanol umkristallisiert, das so erhaltene kristalline DMT abzieht, aus der Mutterlauge das Methanol abdampft und den Rückstand in die Oxidationsstufe zurückführt.

## Claims

1. A process for separating dimethyl isophthalate (DMI) and dimethyl orthophthalate (DMO) and other by-products from their mixture with dimethyl terephthalate (DMT), formed in the oxidation of p-xylene followed by esterification with methanol
a) the esterification product being distilled once or several times,
b) the DMT-rich fraction obtained in step a) being recrystallized from methanol,
c) the crystalline DMT obtained in step b) being taken off, and
d) the methanol being evaporated from the mother liquor of step b),
which comprises
e) melting the residue obtained in step d) and composed mainly of DMT, DMI and DMO and causing DMT to crystallize out by cooling, without the DMT content in the by-products reaching the eutectic composition,
f) taking off the DMT obtained in step e), and
g) at least partially removing the DMI-DMO-fraction, obtained in step e), from the system.

2. The process as claimed in claim 1, wherein, in step e), the molten residue is first cooled within 0.5 to 2 hours to a temperature which is 0.5 to 15 °C below the solidification point of the mixture, the crystals are then allowed to grow and cooling is then taken further, but without reaching the eutectic temperature.

3. The process as claimed in claim 1 or 2, wherein, in step e) cooling is taken down to a temperature which is only 0.5 to 3 °C above the eutectic temperature.

4. The process as claimed in any of claims 1 to 3, wherein, in step g), 5–15% by weight of the DMI-

DMO-fraction is removed from the system and the remainder is recycled into the oxidation stage.

5. A process for separating dimethyl isophthalate (DMI) and dimethyl orthophthalate (DMO) and other by-products from their mixture with dimethyl terephthalate (DMT), formed in the oxidation of p-xylene followed by esterification with methanol
a) the esterification product being distilled once or several times,
b) the DMT-rich fraction obtained in step a) being recrystallized from methanol,
c) the crystalline DMT obtained in step b) being taken off, and
d) the methanol being evaporated from the mother liquor of step b),
which comprises
e) melting a part of the residue obtained in step d) and composed mainly of DMT, DMI and DMO, and causing DMT to crystallize out by cooling, without the DMT content in the by-products reaching the eutectic composition,
f) taking off the DMT obtained in step e),
g) removing the DMI-DMO-fraction, obtained in step e), from the system, and
h) recycling the part, not used in step e), of the residue obtained in step d) into the oxidation stage.

6. The process as claimed in claim 5, wherein, in step e), the molten part of the residue is first cooled within 0.5 to 2 hours to a temperature which is 0.5 to 15 °C below the solidification point of the mixture, the crystals are then allowed to grow and cooling is then taken further, but without reaching the eutectic temperature.

7. The process as claimed in claim 5 or 6, wherein, in step e) cooling is taken down to a temperature which is only 0.5 to 3 °C above the eutectic temperature.

8. The process as claimed in any of claims 5 to 7, wherein 5–15% by weight of the residue obtained in step d) is used in step e) and the remainder is recycled into the oxidation stage.

9. A process for separating dimethyl isophthalate (DMI) and dimethyl orthophthalate (DMO) and other by-products from their mixture with dimethyl terephthalate (DMT), formed in the oxidation of p-xylene followed by esterification with methanol
a) the esterification product being distilled once or several times,
which comprises
b) melting the DMT-rich fraction obtained in step a) and causing DMT to crystallize out by cooling, without the DMT content in the by-products reaching the eutectic composition,
c) recrystallizing the DMT obtained in step b) from methanol,
d) at least partially removing the DMI-DMO-fraction, obtained in step b), from the system,
e) taking off the crystalline DMT obtained in step c), and
f) evaporating the methanol from the mother liquor of step c) and recycling the residue into the oxidation stage.

10. The process as claimed in claim 9, wherein, in step b), the molten DMT-rich fraction is first cooled within 0.5 to 2 hours to a temperature which is 0.5 to 15 °C below the solidification point of the mixture, the crystals are then allowed to grow and cooling is then taken further, but without reaching the eutectic temperature.

11. The process as claimed in claim 9 or 10, wherein, in step b) cooling is taken down to a temperature which is only 0.5 to 3 °C above the eutectic temperature.

12. The process as claimed in any of claims 9 to 11, wherein, in step d), 5–15% by weight of the DMI-DMO-fraction is removed from the system and the remainder is recycled into the oxidation stage.

13. A process for separating dimethyl isophthalate (DMI) and dimethyl orthophthalate (DMO) and other by-products from their mixture with dimethyl terephthalate (DMT), formed in the oxidation of p-xylene followed by esterification with methanol
a) the esterification product being distilled once or several times,
which comprises
b) melting a part of the DMT-rich fraction obtained in step a) and causing DMT to crystallize out by cooling, without the DMT content in the by-products reaching the eutectic composition,
c) recrystallizing the DMT obtained in step b) from methanol,
d) removing the DMI-DMO-fraction, obtained in step b), from the system,
e) taking off the crystalline DMT obtained in step c),
f) evaporating the methanol from the mother liquor of step c) and recycling the residue into the oxidation stage, and
g) recrystallizing the part, not used in step b), of the DMT-rich fraction obtained in step a) from methanol, taking off the crystalline DMT thus obtained, evaporating the methanol from the mother liquor and recycling the residue into the oxidation stage.

14. The process as claimed in claim 13, wherein, in step b), the molten part of the DMT-rich fraction is first cooled within 0.5 to 2 hours to a temperature which is 0.5 to 15 °C below the solidification point of the mixture, the crystals are then allowed to grow and cooling is then taken further, but without reaching the eutectic temperature.

15. The process as claimed in claim 13 or 14, wherein, in step b) cooling is taken down to a temperature which is only 0.5 to 3 °C above the eutectic temperature.

16. The process as claimed in any of claims 13 to 15, wherein 5–15% by weight of the DMT-rich fraction obtained in step a) is used in step b) and the remainder is recycled into the oxidation stage.

17. A process for separating dimethyl isophthalate (DMI) and dimethyl orthophthalate (DMO) and other by-products formed in the oxidation dimethyl terephthalate (DMT), formed in the oxidation of p-xylene and methyl p-toluate followed by esterification with methanol

a) the esterification product being distilled once or several times,
b) the DMT-rich fraction obtained in step a) being recrystallized from methanol,
c) the crystalline DMT obtained in step b) being taken off, and
d) the methanol being evaporated from the mother liquor of step b),
which comprises
e) melting the residue obtained in step d) and composed mainly of DMT, DMI and DMO and causing DMT to crystallize out by cooling, without the DMT content in the by-products reaching the eutectic composition,
f) taking off the DMT obtained in step e), and
g) at least partially removing the DMI-DMO-fraction, obtained in step e), from the system.

18. A process for separating dimethyl isophthalate (DMI) and dimethyl orthophthalate (DMO) and other by-products from their mixture with dimethyl terephthalate (DMT), formed in the oxidation of p-xylene and methyl p-toluate followed by esterification with methanol,
a) the esterification product being distilled once or several times,
b) the DMT-rich fraction obtained in step a) being recrystallized from methanol,
c) the crystalline DMT obtained in step b) being taken off, and
d) the methanol being evaporated from the mother liquor of step b),
which comprises
e) melting a part of the residue obtained in step d) and composed mainly of DMT, DMI and DMO, and causing DMT to crystallize out by cooling, without the DMT content in the by-products reaching the eutectic composition,
f) taking off the DMT obtained in step e),
g) removing the DMI-DMO-fraction, obtained in step e), from the system, and
h) recycling the part, not used in step e), of the residue obtained in step d) into the oxidation stage.

19. A process for separating dimethyl isophthalate (DMI) and dimethyl orthophthalate (DMO) and other by-products from their mixture with dimethyl terephthalate (DMT), formed in the oxidation of p-xylene and methyl p-toluate followed by esterification with methanol
a) the esterification product being distilled once or several times,
which comprises
b) melting the DMT-rich fraction obtained in step a) and causing DMT to crystallize out by cooling, without the DMT content in the by-products reaching the eutectic composition,
c) recrystallizing the DMT obtained in step b) from methanol,
d) at least partially removing the DMI-DMO-fraction, obtained in step b), from the system,
e) taking off the crystalline DMT obtained in step c), and
f) evaporating the methanol from the mother liquor of step c) and recycling the residue into the oxidation stage.

20. A process for separating dimethyl isophthalate (DMI) and dimethyl orthophthalate (DMO) and other by-products from their mixture with dimethyl terephthalate (DMT), formed in the oxidation of p-xylene and methyl p-toluate followed by esterification with methanol
a) the esterification product being distilled once or several times,
which comprises
b) melting a part of the DMT-rich fraction obtained in step a) and causing DMT to crystallize out by cooling, without the DMT content in the by-products reaching the eutectic composition,
c) recrystallizing the DMT obtained in step b) from methanol,
d) removing the DMI-DMO-fraction, obtained in step b), from the system,
e) taking off the crystalline DMT obtained in step c),
f) evaporating the methanol from the mother liquor of step c) and recycling the residue into the oxidation stage, and
g) recrystallizing the part, not used in step b), of the DMT-rich fraction obtained in step a) from methanol, taking off the crystalline DMT thus obtained, evaporating the methanol from the mother liquor and recycling the residue into the oxidation stage.

**Revendications**

1. Procédé pour séparer l'isophtalate de diméthyle (DMI) et l'orthophtalate de diméthyle (DMO) et d'autres sous-produits de leur mélange avec le téréphtalate de diméthyle (DMT), qui a été formé lors de l'oxydation du p-xylène et l'estérification subséquente par le méthanol, procédé selon lequel
a) on distille une ou plusieurs fois le produit de l'estérification,
b) on fait recristalliser à partir de méthanol la fraction, riche en DMT, obtenue à l'étape a),
c) on soutire le DMT cristallin obtenu à l'étape b),
d) on évapore le méthanol de la liquer mère provenant de l'étape b),
procédé caractérisé en ce que:
e) on fait fondre le résidu obtenu à l'étape d), et consistant essentiellement en DMT, DMI et DMO, et par refroidissement, on laisse se séparer par cristallisation DMT, sans parvenir, avec la teneur en DMT des sous-produits, à la composition eutectique,
f) on soutire le DMT obtenu à l'étape e),
g) on enlève au moins partiellement la fraction contenant DMI et DMO, obtenue à l'étape e).

2. Procédé selon la revendication 1, caractérisé en ce que, dans l'étape e), on refroidit le résidu fondu, tout d'abord en l'espace de 0,5 à 2 heures à une température se situant à 0,5 jusqu'à 15 °C au-dessous du point de solidification, puis on laisse croître les cristaux et l'on refroidit ensuite à nouveau, mais sans parvenir à la température eutectique (ou température de formation de l'eutectique).

3. Procédé selon la revendication 1 ou 2, caractérisé en ce que, dans l'étape e), on refroidit jusqu'à une température qui ne se situe qu'à 0,5 à 3 °C au-dessus de la température de l'eutectique.

4. Procédé selon l'une des revendications 1 à 3, caractérisé en ce qu'on élimine dans l'étape g) 5 à 15% en poids de la fraction contenant DMI et DMO et en ce qu'on recycle le reste vers l'étape d'oxydation.

5. Procédé pour séparer l'isophtalate de diméthyle (DMI) et l'orthophtalate de diméthyle (DMO) et d'autres sous-produits de leur mélange avec le téréphtalate de diméthyle (DMT), qui est formé lors de l'oxydation du p-xylène et de l'estérification subséquente par le méthanol, precédé selon lequel

a) on distille une ou plusieurs fois le produit de l'estérification,

b) on soumet à recristallisation, à partir du méthanol, la fraction riche en DMT obtenue à l'étape a),

c) on soutire le DMT cristallin obtenu à l'étape b),

d) on évapore le méthanol de la liqueur mère provenant de l'étape b), procédé caractérisé en ce que

e) on fait fondre une partie du résidu, obtenu à l'étape d), et consistant essentiellement en DMT, DMI et DMO, et par refroidissement, on laisse se séparer par cristallisation, sans parvenir, avec la teneur en DMT des sous-produits, à la composition eutectique,

f) on soutire DMT obtenu à l'étape e),

g) on exclut la fraction contenant DMI et DMO obtenue à l'étape e),

h) on recycle vers l'étape d'oxydation la partie du résidu, obtenue dans l'étape d) et non utilisées à l'étape e).

6. Procédé selon la revendication 5, caractérisé en ce qu'on refroidit la partie du résidu, fondue à l'étape e) tout d'abord en 0,5 à 2 heures jusqu'à une température qui se situe à 0,5 jusqu'à 15 °C au-dessous du point de solidification du mélange, puis on laisse croître les cristaux et l'on refroidit encore à nouveau sans cependant atteindre la température de l'eutectique.

7. Procédé selon la revendication 5 ou 6, caractérisé en ce que, dans l'étape e), on refroidit jusqu'à une température qui ne se situe qu'à 0,5 à 3 °C au-dessus de la température de l'eutectique.

8. Procédé selon l'une des revendications 5 à 7, caractérisé en ce qu'on utilise dans l'étape e) 5 à 15% en poids du résidu obtenu à l'étape d), et l'on recycle le reste vers l'étape d'oxydation.

9. Procédé pour séparer l'isophtalate de diméthyle (DMI) et l'orthophtalate de diméthyle (DMO) et d'autres sous-produits de leur mélange avec le téréphtalate de diméthyle (DMT), qui est formé lors de l'oxydation du p-xylène et de l'estérification subséquente avec le méthanol, precédé selon lequel

a) on distille une ou plusieurs fois le produit de l'estérification, procédé caractérisé en ce que:

b) on fait fondre la fraction riche en DMT, obtenue à l'étape a), et, par refroidissement, on laisse DMT se séparer par cristallisation sans parvenir, avec la teneur en DMT des sous-produits, à la composition eutectique,

c) on fait recristalliser à partir de méthanol le DMT obtenu à l'étape b),

d) on élimine au moins partiellement la fraction de DMI/DMO obtenue à l'étape b),

e) on soutire le DMT cristallin obtenue à l'étape c),

f) on évapore le méthanol de la liqueur mère de l'étape c) et l'on recycle le résidu vers l'étape d'oxydation.

10. Procédé selon la revendication 9, caractérisé en ce que, dans l'étape b), on refroidit la fraction fondue, riche en DMT, tout d'abord en 0,5 à 2 heures jusqu'à une température se situant à 0,5 jusqu'à 15 °C au-dessous du point de solidification du mélange, puis on laisse croître les cristaux et l'on refroidit ensuite à nouveau, sans cependant atteindre la température de l'eutectique.

11. Procédé selon la revendication 9 ou 11, caractérisé en ce que, dans l'étape b), on refroidit jusqu'à une température qui ne se situe qu'à 0,5 jusqu'à 3 °C au-dessus de la température de l'eutectique.

12. Procédé selon l'une des revendications 9 à 11, caractérisé en ce que, dans l'étape d), on élimine 5 à 15% en poids de la fraction DMI-DMO et l'on recycle le reste vers l'étape d'oxydation.

13. Procédé pour séparer l'isophtalate de diméthyle (DMI) et l'orthophtalate de diméthyle (DMO) ainsi que d'autres sous-produits de leur mélange avec le téréphtalate de diméthyle (DMT), qui a été formé lors de l'oxydation du p-xylène et l'estérification subséquente par le méthanol, procédé selon lequel:

a) on distille une ou plusieurs fois le produit de l'estérification, procédé caractérisé en ce que:

b) on fait fondre une partie de la fraction, riche en DMT, obtenue à l'étape a) et, par refroidissement, on laisse DMT se séparer par cristallisation sans parvenir, avec la teneur en DMT des sous-produits, à la composition de l'eutectique,

c) on fait recristalliser à partir de méthanol le DMT obtenu dans l'étape b),

d) on élimine la fraction de DMI-DMO obtenue à l'étape b),

e) on soutire le DMT cristallin obtenue à l'étape c),

f) on évapore le méthanol de la liqueur mère de l'étape c) et l'on recycle le résidu vers l'étape d'oxydation,

g) on fait recristalliser à partir de méthanol la partie, non utilisée à l'étape b) de la fraction riche en DMT obtenue à l'étape a), on soutire le DMT cristallin ainsi obtenu, on évapore le méthanol de la liqueur mère et l'on recycle le résidu vers l'étape d'oxydation.

14. Procédé selon la revendication 13, caractérisé en ce que, dans l'étape b), on refroidit la partie fondue de la fraction riche en DMT tout d'abord, en 0,5 à 2 heures, jusqu'à une température se situant à 0,5 jusqu'à 15 °C au-dessous du point de solidification du mélange, puis on laisse croître les cristaux et l'on refroidit ensuite à nouveau, sans cependant parvenir à la température de l'eutectique.

15. Procédé selon la revendication 13 ou 14, caractérisé en ce que, dans l'étape b), on refroidit jusqu'à une température qui ne se situe qu'à 0,5 à 3 °C au-dessus de la température de formation de l'eutectique (température de l'eutectique).

16. Procédé selon l'une des revendications 13 à 15, caractérisé en ce que, dans l'étape b), on utilise 5 à 15% en poids de la fraction, riche en DMT, obtenue à l'étape a), et l'on recycle le reste vers l'étape d'oxydation.

17. Procédé pour séparer l'isophtalate de diméthyle (DMI) et l'orthophtalate de diméthyle (DMO) et d'autres sous-produits de leur mélange avec le téréphtalate de diméthyle (DMT), qui a été formé lors de l'oxydation d'un mélange de p-xylène et d'ester méthylique de l'acide p-toluylique puis estérification avec le méthanol, procédé selon lequel:
a) on distille une ou plusieurs fois le produit de l'estérification,
b) on fait recristalliser à partir de méthanol la fraction, riche en DMT, obtenue à l'étape a),
c) on soutire le DMT cristallin obtenu à l'étape b),
d) on évapore le méthanol de la liqueur mère provenant de l'étape b),
procédé caractérisé en ce que:
e) on fait fondre le résidu, obtenu dans l'étape d), et consistant surtout en DMT, DMI et DMO, et, par refroidissement, on laisse DMT se séparer par cristallisation, sans parvenir, avec la teneur en DMT des sous-produits, à la composition eutectique,
f) on soutire le DMT obtenu dans l'étape e),
g) on exclut au moins partiellement la fraction de DMI-DMO obtenue à l'étape e).

18. Procédé pour séparer l'isophtalate de diméthyle (DMI) et l'orthophtalate de diméthyle (DMO) et d'autres sous-produits de leur mélange avec le téréphtalate de diméthyle (DMT), qui a été formé lors de l'oxydation d'un mélange de p-xylène et de l'ester méthylique de l'acide p-toluylique puis estérification à l'aide de méthanol, procédé selon lequel:
a) on distille une ou plusieurs fois le produit de d'estérification,
b) on fait recristalliser, partir du méthanol, la fraction riche en DMT, obtenue à l'étape a),
c) on soutire le DMT cristallin obtenu à l'étape b),
d) on évapore le méthanol de la liqueur mère provenant de l'étape b),
procédé caractérisé en ce que:
e) on fait fondre une partie du résidu, obtenu à l'étape d), et consistant surtout en DMT, DMI et DMO, et, par refroidissement, on laisse DMT se séparer par cristallisation, sans parvenir, avec la teneur en DMT des sous-produits, à la composition eutectique,
f) on soutire le DMT obtenu à l'étape e),

g) on élimine la fraction de DMI-DMO obtenue à l'étape e),
h) on recycle vers l'étape d'oxydation la partie, non utilisée à l'étape e), du résidu obtenu á l'étape d).

19. Procédé pour séparer l'isophtalate de diméthyle (DMI) et l'orthophtalate de diméthyle (DMO) et d'autres sous-produits, de leur mélange avec le téréphtalate de diméthyle (DMT), qui a été formé lors de l'oxydation d'un mélange de p-xylène et de l'ester méthylique de l'acide p-toluylique puis estérification par le méthanol, procédé selon lequel:
a) on distille une ou plusieurs fois le produit de l'estérification,
procédé caractérisé en ce que:
b) on fait fondre la fraction, riche en DMT, obtenue à l'étape a) et, par refroidissement, on laisse DMT se séparer par cristallisation, sans parvenir, avec la teneur en DMT des sous-produits, à la compositions eutectique,
c) on fait recristalliser à partir de méthanol le DMT obtenu à l'étape b),
d) on élimine, au moins partiellement, la fraction de DMI/DMO obtenue à l'étape b),
e) on soutire le DMT cristallin obtenu à l'étape c),
f) on évapore le méthanol de la liqueur mère l'étape c) et l'on recycle le résidu vers l'étape d'oxydation.

20. Procédé pour séparer l'isophtalate de diméthyle (DMI) et l'orthophtalate de diméthyle (DMO) et d'autres sous-produits de leur mélange avec le téréphtalate de diméthyle (DMT), qui a été formé lors de l'oxydation d'un mélange de p-xylène et de l'ester méthylique de l'acide p-toluylique puis estérification avec le méthanol, procédé selon lequel:
a) on distille une ou plusieurs fois le produit de l'estérification,
procédé caractérisé en ce que:
b) on fait fondre une partie de la fraction, riche en DMT, obtenue à l'étape a) et, par refroidissement, on laisse DMT se séparer par cristallisation, sans parvenir, avec la teneur en DMT des sous-produits, à la composition de l'eutectique,
c) on fait recristalliser à partir de méthanol le DMT obtenu à l'étape b),
d) on élimine la fraction de DMI/DMO obtenue à l'étape b),
e) on soutire le DMT cristallin obtenu à l'étape c),
f) on évapore le méthanol de la liqueur mère provenant de l'étape c) et l'on recycle le résidu vers l'étape d'oxydation,
g) on fait recristalliser à partir de méthanol la partie, non utilisée à l'étape b), de la fraction, riche en DMT, obtenue à l'étape a), on soutire le DMT cristallin ainsi obtenu, on évapore le méthanol de la liqueur mère et l'on recycle le résidu vers l'étape d'oxydation.